# EUROPEAN PATENT APPLICATION

(11) **EP 4 309 603 A1**
(43) Date of publication of application: **24.01.2024**
(21) Application number: 22839112.4
(22) Date of filing: 04.08.2022
(51) Int. Cl.: A61B 18/00, A61B 18/12, A61B 18/14

(54) **RADIO FREQUENCY ABLATION APPARATUS**

(30) Priority: 25.05.2022 CN 202210585407; 25.05.2022 CN 202221277599 U
(71) Applicant: LEPU MEDICAL TECHNOLOGY (BEIJING) CO., LTD., Beijing 102200 (CN)
(72) Inventor: WANG, Lei, Beijing 102200 (CN); DING, Yishou, Beijing 102200 (CN); ZHANG, Yuxin, Beijing 102200 (CN); PU, Zhongjie, Beijing 102200 (CN); HE, Tao, Beijing 102200 (CN); LIU, Qi, Beijing 102200 (CN)
(74) Representative: J A Kemp LLP
(86) International application number: PCT/CN2022/110279
(87) International publication number: WO 2023/226194

(57) **Abstract**

A radiofrequency ablation device comprising an electrode, a balloon and a delivery tube; the electrode is in a shape of a mesh which has a contracted state that shrinks together under restriction and an expanded state that automatically expands outward without restriction, and is connected to an external electrical connector through an electrode lead; the balloon is located at the inner side of the electrode, and pushes against the electrode when it is in an expanded state; the delivery tube comprises an outer tube and an inner tube, the electrode lead is located between the electrical outer tube and the inner tube, and the delivery tube further comprises a balloon catheter communicating with one end of the balloon, which is located at the inner side of the inner tube and has a gap with the inner tube. When the electrode is delivered to the target position, it can expand automatically and form a good abutment effect with the inner wall of the bronchus by means of the support of the balloon.

## Description

### TECHNICAL FIELD

The invention relates to the technical field of tissue ablation, in particular to a radiofrequency ablation device.

### BACKGROUND TECHNIQUE

Chronic Obstructive Pulmonary Disease (COPD) is a general term for diseases that cause obstruction of airflow in the airways or respiratory tract, specifically such as chronic bronchitis and/or emphysema. Such a disease is chronic, its airflow obstruction is often permanent or irreversible, and it can progress to common chronic diseases such as pulmonary heart disease and/or respiratory failure.

The main treatment of COPD is to relieve current symptoms and reduce future risks. Traditional treatment methods are to control the living environment and use means such as drug therapy, oxygen therapy and ventilation support. In severe cases, lung resection is required.

Using drugs to prevent and control symptoms indeed reduces the frequency of acute and major morbidity, thereby improving exercise tolerance and quality of life. However, patients need to persist in taking the drugs for a long time, which will cause great physical and economic pressure to patients.

With the help of surgical treatment methods, such as bulla resection, lung volume reduction (removal of part of the lung tissue), bronchoscopy lung volume reduction and lung transplantation, the pain of long-term medication can be alleviated and the economic pressure of patients can be reduced, but the surgical trauma is large, and the patients suffer too much pain during the treatment.

In short, with the existing treatment methods, patients suffer great pain, such as the pain of taking medicine for a long time and the pain caused by the large surgical trauma.

The prior art disclosed a system, device, and method for treating tissue and controlling stenosis, comprising an ablation assembly, wherein an ablation assembly comprises a balloon and a wave-shaped electrode attached to the outside of the balloon.

During operation, the balloon is inflated to expand and the wave-shaped electrode attached to the outside of the balloon is expanded to abut against the diseased tissue on the inner wall of the bronchus, and perform radiofrequency ablation on it.

However, this kind of ablation assembly has the following disadvantages: since the wave-shaped electrode is attached to the outside of the capsule, it cannot expand automatically to abut against the inner wall of the bronchus, and it requires the support of a balloon to make contact with the inner wall of the bronchus and thus achieve the ablation of the diseased tissue.

### SUMMARY OF THE INVENTION

Therefore, the technical problem to be solved in this application is to overcome the defect that the electrode in the prior art cannot expand automatically to abut against the inner wall of the bronchus, and a radiofrequency ablation device in which the electrode can expand automatically and abut against the inner wall of the bronchus is thus provided.

To solve the above problem, this application provides a radiofrequency ablation device which comprises:
an electrode in a shape of a mesh, which has a contracted state that shrinks together under restriction and an expanded state that automatically expands outward without restriction, and is connected to an external electrical connector through an electrode lead;
a balloon located at the inner side of the electrode, which pushes against the electrode when it is in an expanded state;
a delivery tube comprising an outer tube and an inner tube, the electrode lead is located between the outer tube and the inner tube, the delivery tube further comprises a balloon catheter communicating with one end of the balloon, which is located at the inner side of the inner tube and has a gap with the inner tube.

Optionally, the electrode is formed by cutting a memory alloy.

Optionally, the electrode is provided with an ablation zone, which is located on a raised part of the electrode.

Optionally, the entire electrode constitutes the ablation zone.

Optionally, a free end of the electrode extends outward.

Optionally, the distance between the free end of the electrode and the central axis of the electrode is smaller than the distance between the raised part and the central axis of the electrode or smaller than a radius of the bronchus.

Optionally, the balloon, when inflated, has a diameter greater than the diameter of the electrode at the point where the balloon abuts against the electrode, which is suitable for pushing the electrode towards the inner wall of the bronchus.

Optionally, the raised part comprises an end of the electrode, and the ablation zone is located on the end of the electrode.

Optionally, the balloon is provided with micropores on its surface.

Optionally, the balloon catheter is provided with an input cavity (42a) adapted to input coolant into the balloon, and an output cavity (42b) adapted to provide a passage for the coolant to flow out of the balloon.

Optionally, the radio frequency ablation device further comprises a sensor arranged on the electrode, wherein the sensor is connected to the electrical connector through a sensor leadwhich is located between the outer tube and the inner tube.

Optionally, the radiofrequency ablation device further comprises a negative plate; a radiofrequency device electrically connected to the negative plate and connected to the electrical connector; a pressure pump connected to the balloon connector through a first butt tube; a circulating pump connected to a coolant connector through a second butt tube; a bronchoscopy device comprising a bronchoscope, an endoscopic visualization device and a suction device, wherein the bronchoscope has a working channel suitable for the passage of the delivery tube.

This application has the following advantages:
1. In the radio frequency ablation device provided by the embodiment of this application, the ablation zone is arranged on the raised part of the electrode, the ablation zone is more closely abutted against the inner wall of the bronchus compared to the ablation zone arranged in other parts, which improves the ablation effect.
2. In the radio frequency ablation device provided by the embodiment of this application, the raised part comprises the end of the electrode, the ablation zone is located at the raised part on the end of the electrode, which can reduce the size of the electrode, thus facilitating the passage of the electrode through the bronchus and improving the passing performance of the electrode.
3. In the radio frequency ablation device provided by the embodiment of this application, the free end of the electrode extends outward, so as to prevent the balloon in the contracted state from being punctured by the free end of the electrode during the process of entering the electrode or the process of moving the balloon relative to the electrode, thereby preventing the failure.
4. In the radio frequency ablation device provided by the embodiments of this application, the distance between the free end of the electrode and the central axis of the electrode is smaller than the distance between the raised part and the center axis of the electrode or smaller than the radius of the bronchus, so as to prevents the inner wall of the bronchus being punctured by the free end of the electrode during the expanding process of the electrode and/or the balloon.
5. In the radio frequency ablation device provided by the embodiment of this application, the electrode lead and the sensor lead are located between the outer tube and the inner tube, so as to prevent the sensor lead and the electrode lead from being involved in the process of use, resulting in poor contact between the sensor lead and the sensor and/or poor contact between the electrode lead and the electrode, which affect the surgical effect, and at the same time, it can make the appearance of the delivery tube neat and tidy, easy to store and organize.
6. In the radio frequency ablation device provided by the embodiments of this application, the electrode is not attached to the balloon. Therefore, during the delivery process, the balloon is delivered to the target position first, and then the electrode is delivered to the target position, the balloon passes through the bronchus, that is, the larger size of the balloon is allowed to pass through the bronchus with the same size, thereby increasing the support force of the balloon to the electrode, and at the same time, more coolant is injected into the balloon to improve its cooling effect on the electrode.
7. In the radiofrequency ablation device provided by the embodiments of this application, because the electrode is in a shape of a mesh which has a contracted state that shrinks together under restriction and an expanded state that automatically expands outward without restriction, so when the electrode is delivered to the target position, it can expand automatically to abut against the inner wall of the bronchus, without relying on the support of the balloon, which can avoid the failure of the balloon and the inability to perform ablation.
8. In the radio frequency ablation device provided by the embodiment of this application, the free end of the electrode is provided with scratch-resistant structures, which can prevent the free end from damaging the balloon when the balloon is retracted.
9. In the radio frequency ablation device provided by the embodiment of this application, each of the anti-scratch structures at the free end of the electrode is provided with a small hole, and a connecting line passes through adjacent small holes in turn to form a spacing ring, which can prevent the shape of the balloon from changing in the body after constrained, such as being formed into a sheet, so that a portion of the balloon may enter the free end of the electrode and then be stucked between the scratch-resistant structures of adjacent electrodes.

### BRIEF DESCRIPTION OF THE DRAWINGS

To more clearly illustrate the specific embodiments of this application or the technical solutions in the prior art, the following briefly describes the accompanying drawings that are required for describing the specific embodiments or the prior art. Obviously, the accompanying drawings described below are some embodiments of this application. A person of ordinary skill in the art may further obtain other accompanying drawings based on the accompanying drawings without creative efforts.
FIG. 1 is a structural schematic diagram of the radiofrequency ablation device according to the embodiment of this application;
FIG. 2 is a partial structural schematic diagram of the radiofrequency ablation device according to the embodiment of this application;
FIG. 3 is a schematic diagram of the cooperation between the balloon and the electrode in the radiofrequency ablation device according to the embodiment of this application;
FIG.4 is a schematic cross-sectional view of the delivery tube in the A-A position according to the embodiment of this application;
FIG. 5 is a schematic diagram of the first embodiment of the electrode according to the embodiment of this application;
FIG. 6 is a schematic diagram of the second embodiment of the electrode according to the embodiment of this application;
FIG. 7 is a schematic diagram of the third embodiment of the electrode according to the embodiment of this application;
FIG. 8 is a schematic diagram of the external structure of the balloon and the balloon catheter according to the embodiment of this application;
FIG. 9 is a schematic diagram of the internal structure of the first embodiment of the balloon and the balloon catheter according to the embodiment of this application;
FIG. 10 is a schematic cross-sectional view of the balloon catheter in the B-B position according to the embodiment of this application;
FIG. 11 is a schematic diagram of the internal structure of the second embodiment of the balloon and the balloon catheter according to the embodiment of this application;
FIG. 12 is a schematic cross-sectional view of the balloon catheter in the C-C position of the embodiment of this application;
FIG. 13 is a schematic diagram of the external structure of the balloon provided with micropores according to the embodiment of this application.

### Descriptions of reference signs:

1. electrode; 10. end; 11. ablation zone; 15. raised part; 16. tail end; 17. free end; 18. anti-scratch structure; 19. spacing ring; 12. balloon; 120. micropore; 121. small hole; 122. pointed head; 13. inner wall of bronchus; 2. bronchus endoscope; 20. endoscopic visualization device; 21. suction device; 3. sensor; 4. delivery tube; 40. outer tube; 41. inner tube; 42. balloon catheter; 42a. input cavity; 42b. output cavity; 44. electrode lead; 45. sensor lead; 5. handle; 50. balloon connector; 51. electrical connector; 52. coolant connector; 6. circulating pump; 60. first butt tube; 61. second butt tube; 7. radiofrequency device; 8. negative plate; 9. pressure pump.

### DETAILES DESCRIPTION

Technical solutions of this application are clearly and completely described below with reference to the accompanying drawings. Obviously, the described embodiments are some, but not all, of the embodiments of this application. Based on the embodiments of this application, all other embodiments obtained by a person of ordinary skill in the art without making creative efforts shall fall within the protection scope of this application.

In the descriptions of this application, it should be noted that an orientation or positional relationship indicated by terms "center", "upper", "lower", "left", "right", "vertical", "horizontal", "inner", "outer", and the like is based on an orientation or positional relationship shown in the accompanying drawings, and is only for the convenience of describing this application and simplifying the descriptions, rather than indicating or implying that the indicated apparatus or component must have a specific orientation or must be constructed and operated in a specific orientation. Therefore, the orientation or positional relationship should not be construed as limitations on this application. Furthermore, terms "first", "second", and "third" are used for descriptive purposes only and should not be construed to indicate or imply relative importance.

In the descriptions of this application, it should be noted that unless otherwise expressly specified and limited, the terms "installed", "connected" and "connected" should be construed broadly, for example, it can be a fixed connection, a detachable connection, or an integral connection; it can be a mechanical connection or an electrical connection; it can be directly connected, or indirectly connected through an intermediate medium, or it can be the internal connection of two components. For those of ordinary skill in the art, the specific meanings of the above terms in this application can be understood according to specific situations.

In addition, the technical features involved in the different embodiments of this application described below can be combined with each other provided there is no conflict with each other.

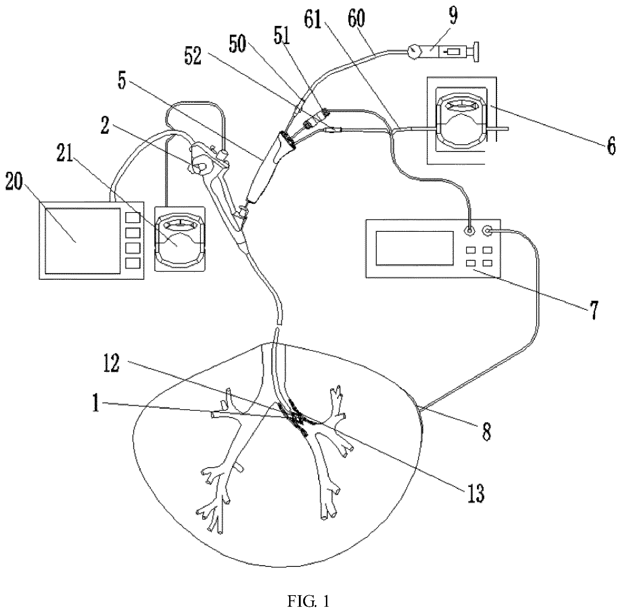

As shown in FIG. 1 to FIG. 13, this embodiment provides a radio frequency ablation device for ablating a diseased tissue in a human body, specifically in this embodiment, for ablating the tissue on the diseased inner wall of the bronchus 13.

The radiofrequency ablation device comprises an electrode 1, a balloon 12 for supporting the electrode, and a sensor 3 arranged on the electrode 1.

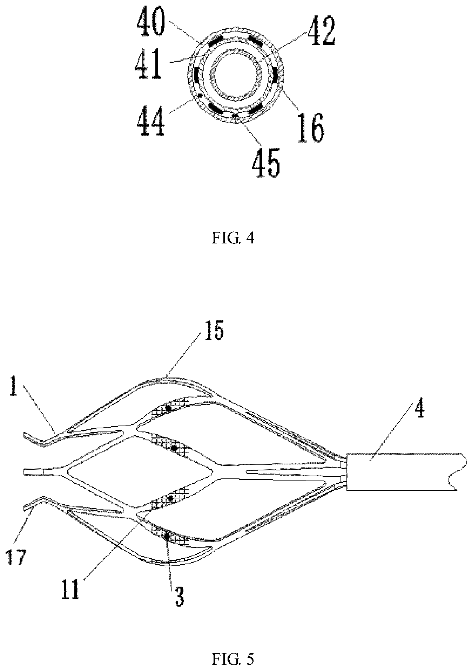

As shown in FIG. 5, after being laser-cut from memory alloy and heat-treated, the electrode 1 is shaped into a mesh. The electrode 1 may also be of other shapes, which are not limited here. The memory alloy can be nickel-titanium alloy, and other alloys with memory function can also be used.

Certainly, the electrode 1 can also use a non-metallic material as the skeleton, and a conductive material is arranged on the skeleton to form the ablation zone 11. The ablation zone 11 can be arranged independently, or can be connected to each other, it can be in a sheet, ring or other form. In this embodiment, the ablation zone 11 is located at the raised part 15 of the electrode 1, which is independently controlled by the device to achieve the purpose of ablation of the entire circumference or ablation of a single point.

Electrode 1 has a contracted state that shrinks together under restriction, and an expanded state that automatically expands outward without restriction, so that, as shown in FIG. 1, when electrode 1 reaches the target position, it can automatically expand and abut against the inner wall of bronchus 13, and no supporting device is required to spread the electrode 1, thereby simplifying the radiofrequency ablation device which employs such a electrode. Certainly, the electrode 1 in this embodiment can also perform radiofrequency ablation on other tissues of human body, which is not limited here.

As shown in FIG. 5, it is the first embodiment of the electrode 1, which is in a shape of a rugby ball, and a plurality of ablation zones 11 are formed on the raised part 15 of the electrode 1. Compared with arranging the ablation zones 11 in the area other than the raised part 15, arranging the ablation zones 11 on the raised part 15 of the electrode 1 can increase the abutment effect between the ablation zones 11 of the electrode 1 and the inner wall of bronchus 13, and the design of multiple ablation zones 11 is convenient to form abutment at multiple sites against the inner wall of bronchus 13 so as to ablate multiple diseased tissues.

In this embodiment, the free end 17 of the electrode 1 extends outward and is smooth, so that the balloon 12 can be prevented from being punctured by the free end 17 of the electrode 1 during the process when the balloon 12 in the contracted state is placed into the electrode 1 or the balloon 12 and the electrode 1 move relative to each other. The distance between the free end 17 of the electrode 1 and the central axis of the electrode 1 is smaller than the distance between the raised part 15 and the central axis of the electrode 1, or, the distance between the free end 17 of the electrode 1 and the central axis of the electrode 1 is smaller than the radius of the inner wall of bronchus 13, so that the inner wall of bronchus 13 is prevented from being scratched by the free end 17 of the electrode 1 during the expanding process of the electrode 1 and/or the balloon 12.

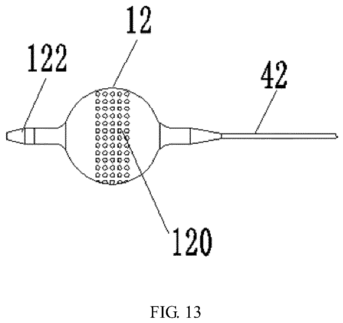

As shown in FIG. 13, which is another embodiment of the electrode 1, the free end 17 of the electrode 1 is no longer in an expanded state, but in a contracted state, and the free end 17 is provided with several anti-scratch structures 18, which can be a smooth ball or an isolated block formed integrally with the electrode 1 or formed separately, and the scratch-resistant structures 18 can also be in the form of rubber caps directly arranged on the free end 17. The function of the anti-scratch structures 18 is to prevent the free end 17 from damaging the balloon 12 when the balloon 12 is retracted. There is a small hole (not marked) in the center of each anti-scratch structure 18 with a soft connecting wire passing through the small hole on each of the adjacent anti-scratch structures 18 to form a spacing ring 19, which restricts the balloon 12 inside the electrode 1 to prevent the balloon 12 from changing in shape after being contracted in the body. For example, when it is formed into the shape of a sheet, a part of the balloon 12 may enter the free end 17 of the electrode 1, and then be caught between the anti-scratch structures 18 of the adjacent electrode 1. The connecting wire is preferably made of high molecular polyethylene material, but certainly other materials can also be used.

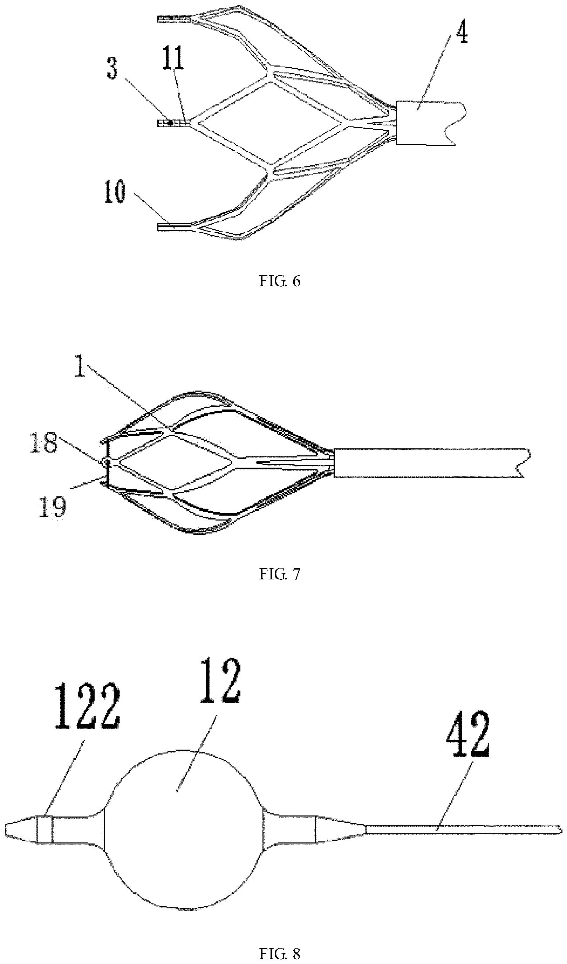

As shown in FIG. 6, it is the second embodiment of the electrode 1, which is in a shape of lotus flower, the raised part 15 of the electrode 1 comprises the end 10 of the electrode 1, the ablation zone 11 is located at the end 10 of the electrode 1, and the end 10 is more expanded outward relative to other parts of the electrode 1, so that after the electrode 1 enters the bronchus, the ablation zones 11 located at the end 10 can better abut against the inner wall of bronchus 13. As the ablation zones 11 are located at the end 10 of the electrode 1 and are located on the raised part, the size of the electrode 1 can be reduced compared to the electrode 1 in the first embodiment, which facilitates the passage of the electrode 1 through the bronchus and improves the passing performance of the electrode.

Certainly, the ablation zone 11 can also be arranged at any position on the electrode 1, and the length of the ablation zones 11 on the electrode 1 can be any length.

In another embodiment, the entire electrode 1 constitutes the ablation zone 11, which can ablate a relatively large amount of diseased tissue at one time, and also simplifies the manufacturing process of the electrode 1. Additionally, Electrode 1 requires only one electrode lead to connect to the external device. Moreover, compared with arranging too many ablation zones 11, the entire electrode 1 constitutes an ablation zone 11, which can avoid the inability to ablate the diseased tissue corresponding to the failed ablation zone 11 on the bronchus due to the failure of a certain ablation zone 11.

The parts of the electrode 1 other than the ablation zone 11 is coated with an insulating layer to prevent leakage or dispersion of radiofrequency energy, and also to prevent damage to other non-pathological tissues. The insulating layer is formed by spray coating or dip coating, and it can be arranged in a form of bonding or in any other existing arrangement.

When not working, the balloon 12 is in a contracted state, and is inflated when in a working state, and the inflated state of the balloon 12 is usually achieved by means of inflation. Certainly, the balloon 12 can also be inflated by injecting coolant into it. In this embodiment, the means of injecting coolant is selected. When the balloon 12 is inflated, its diameter at the place where it abuts against the electrode 1 is greater than the diameter of the electrode 1 at the place where the electrode 1 is abutted by the balloon 12, so that the balloon 12 pushes against the electrode 1 when it is in the expanded state, so that the electrode 1 is pushed toward the inner wall of bronchus 13, which further enhances the abutment effect between the electrode 1 and the inner wall of bronchus 13. The balloon 12 can be cylindrical, spherical, gourd-shaped or other shapes, the part supporting the electrode 1 is adapted to the inner shape of the electrode 1, and the material of the balloon 12 is preferably a material with good compliance such as TPU.

As shown in FIG. 5 and FIG. 6, the sensor 3 is arranged on the electrode 1, it can be a temperature sensor or a pressure sensor, and there may be one or more sensors 3. The temperature sensor and the pressure sensor can be arranged independently on the electrode 1. The sensor 3 can also be an integrated sensor, which has both functions of temperature detection and pressure detection. The integrated sensor is sandwiched between the electrode 1 and the inner wall of bronchus 13 during use, which monitors the temperature of the electrode 1 and the inner wall of bronchus 13, and the pressure between the electrode 1 and the inner wall of bronchus 13, such that the abutment effect between the two can be better judged.

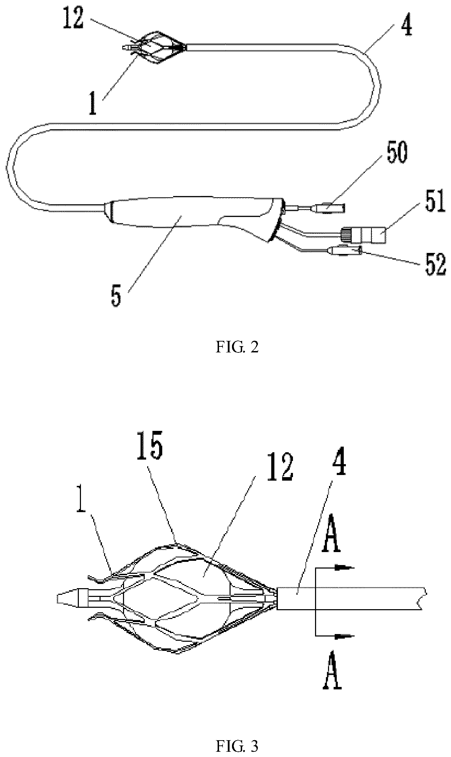

As shown in FIG. 3 and FIG. 4, the radiofrequency ablation device further comprises a delivery tube 4. The electrode 1, the balloon 12 and the sensor 3 are all connected to an external device through the delivery tube 4. The delivery tube 4 sequentially comprises an outer tube 40, an inner tube 41 and a balloon catheter 42 from outside to inside. From the perspective of FIG. 4, the balloon catheter 42 is located at the innermost side and has a gap with the inner tube 41.

In addition to delivering gas, the balloon catheter 42 can also deliver coolant. In this embodiment, the balloon catheter 42 delivers coolant. The coolant may specifically be cooling water, cooling brine, or other fluids with lower temperature.

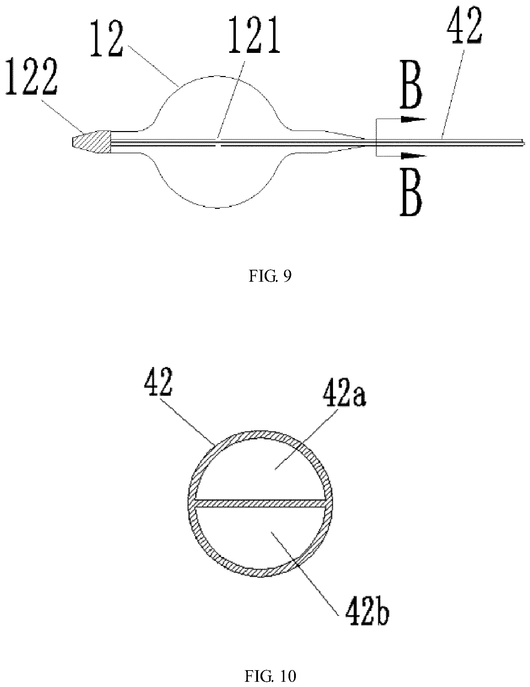

As shown in FIG. 3 and FIG. 9, the balloon catheter 42 extends out of the delivery tube 4, enters the balloon 12, and extends from one end of the balloon 12 to the other end, so that the balloon 12 can provide support for the electrode 1. While the balloon catheter 42 provides support for the balloon 12, so as to prevent the balloon 12 from falling and deforming after the coolant enters the balloon 12, which affects the supporting effect for the electrode 1. The distal end of the balloon catheter 42 is provided with a small hole 121 to communicate with the balloon 12. After the coolant enters the balloon catheter 42, it enters the balloon 12 through the small hole 121 to reduce the impact on the balloon 12.

As shown in FIG. 9 and FIG. 10, it is the first embodiment of the balloon catheter 42, a partition plate is arranged in the middle to divide the balloon catheter 42 into an input cavity 42a and an output cavity 42b. The coolant enters the balloon 12 from the input cavity 42a and flows out of the balloon 12 from the output cavity 42b.

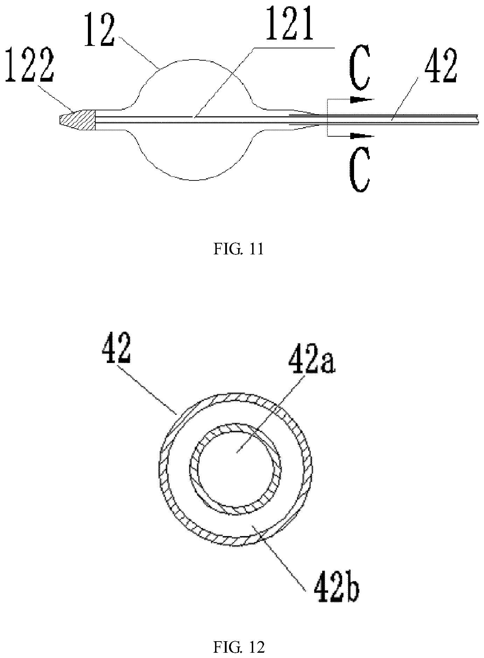

As shown in FIG. 11 and FIG. 12, it is the second embodiment of the balloon catheter 42, which is a double-layer tube, so as to form an input cavity 42a and an output cavity 42b. The coolant enters the balloon 12 from the input cavity 42a and flows out of the balloon 12 from the output cavity 42b.

As the balloon catheter 42 is provided with an output cavity 42b, after the coolant enters the balloon 12 and circulates, it flows out of the balloon 12 through the output cavity 42b, and the coolant does not enter the human body, so as to avoid stimulating the nerves of the airway tissue, causing discomfort to the human body and affecting the treatment. At the same time, there is no need to extract coolant during the operation so as to shorten the operation time.

As shown in FIG. 4, there are an electrode lead 44 and a sensor lead 45 arranged between the outer tube 40 and the inner tube 41, so as to prevent the thin electrode lead 44 and the sensor lead 45 from being involved during use, which may lead to the poor contact between the electrode lead 44 and the electrode 1 and/or the poor contact between the sensor lead 45 and the sensor 3, thus affecting the surgical effect. At the same time, the appearance of the delivery tube 4 can be neat and easy to be stored and organized. One end of the electrode lead 44 is connected to the tail end 16 of the electrode 1, and the tail end 16 of the electrode 1 enters between the outer tube 40 and the inner tube 41, so that the electrode 1 can be better stabilized.

As shown in FIG. 2, the radiofrequency ablation device further comprises a handle 5 connecting to the delivery tube 4, and the handle 5 provides a gripping position for the operator to facilitate the operator to push and/or rotate the delivery tube 4, the electrode 1 and the balloon 12 to adjust the ablation position.

The handle 5 comprises a balloon connector 50, an electrical connector 51, and a coolant connector 52.

The balloon connector 50 is connected to the input cavity 42a of the balloon catheter 42, the electrical connector 51 is connected to the electrode lead 44, the electrical connector 51 is also connected to the sensor lead 45, and the coolant connector 52 is connected to the output cavity 42b of the balloon catheter 42.

The radiofrequency ablation device further comprises a pressure pump 9 and a circulation pump 6. The pressure pump 9 is connected to the balloon connector 50 through the first butt tube 60, and the circulation pump 6 is connected to the coolant connector 52 through the second butt tube 61.

As shown in FIG. 13, which is another embodiment of the balloon, the surface of the balloon 12 is provided with micropores 120, which are uniformly or non-uniformly distributed around the balloon 12, or only arranged in the area contacting with the electrode 1. Since the micropores 120 provided on the surface of the balloon 12 provide a passage for the coolant to flow out of the balloon 12, the output cavity 42b is no longer provided in the balloon catheter 42. The coolant flows to the electrode 1 and the inner wall of bronchus 13 through the micropores 120 to cool and protect the electrode 1 and the tissue on the inner wall of bronchus 13. Since the coolant can cool the inner wall of bronchus 13, it can increase the depth of ablation and enhance the ablation effect.

As another embodiment, by controlling the total area of the micropores 120 on the balloon 12, the coolant can be discharged out of the balloon 12 through the micropores 120 and part of the coolant can flow out through the output cavity 42b of the balloon catheter 42.

The radiofrequency ablation device further comprises a radiofrequency device 7 and a negative plate 8. The radiofrequency device 7 is connected to the negative plate 8, and at the same time, the radio frequency device 7 is also electrically connected to an electrical connector 51. Thus, the electrode 1 is connected to the radiofrequency device 7 through the electrode lead 44 and the electrical connector 51, and the sensor 3 is connected to the radiofrequency device 7 through the sensor lead 45 and the electrical connector 51.

The radiofrequency ablation device further comprises a bronchoscope device (not marked) which comprises a bronchus endoscope 2, an endoscopic visualization device 20 and a suction device 21. The bronchus endoscope 2 provides access to the bronchus for the delivery tube 4, the endoscopic visual device 20 provides images to guide the delivery tube 4 and the electrode 1 and the balloon 12 connected to the delivery tube 4 to achieve the treatment site, and the suction device 21 suctions the coolant injected into the bronchi during the operation.

In the following, the specific process of using the radio frequency ablation device provided in this embodiment will be described:
during use, the negative plate 8 is installed on the skin of a patient, and the negative plate 8 is connected to the radiofrequency device 7, and the bronchus endoscope 2 is delivered to the target site in the bronchus through the human oral cavity;
the electrode 1, the balloon 12 and the sensor 3 connected to the delivery tube 4 are delivered to the target site along the working channel of the bronchus endoscope 2 with the help of the delivery tube 4. During the delivery, the endoscope image is used to determine whether the electrode 1 is in place. At the same time, during the delivery, the electrode 1 is constrained in a contracted state by the inner wall of the working channel, and the balloon 12 is also in a contracted state so as to pass through the working channel. After the electrode 1 reaches the target position and extends out of the bronchus endoscope 2, the electrode 1 is expanded into a mesh shape, and the expanded electrode 1 is fitted with the inner wall of bronchus 13;
the balloon connector 50 on the handle 5 is connected to the pressure pump 9 through the first butt tube 60, the electrical connector 51 on the handle 5 is connected to the radiofrequency device 7, and the coolant connector 52 is connected to the circulation pump 6 through the second butt tube 61;
the pressure pump 9 is started to inject coolant into the balloon 12 through the first butt tube 60, the balloon connector 50, the input cavity 42a of the balloon catheter 42 and the small hole 121 at the distal end of the balloon catheter 42, so that the balloon 12 is in an expanded state, thus further forcing the electrode 1 to fit more closely with the inner wall of bronchus 13;
the radiofrequency device 7 is stated, at which a complete radiofrequency loop is formed among the electrode 1 - the inner wall of bronchus 13 - the human body - the negative plate 8 - the radiofrequency device 7, the radiofrequency current enters the inner wall of bronchus 13 through the electrode 1, and the rapid change of the electromagnetic field makes the positive and negative ions in the cells at the inner wall move rapidly, so the friction between them and other molecules and ions in the adjacent cells causes the lesion to warm up rapidly, causing the water inside and outside the cells to evaporate, dry, shrink, and shed, so as to achieve the purpose of treatment, and the heat is transferred to the electrode 1, and the electrode 1 also becomes hot;
when the radiofrequency device 7 is started, the circulation pump 6 is started at the same time, and the coolant flows into the circulation pump 6 through the output cavity 42b, the coolant connector 52, and the second butt tube 61 after circulating within the balloon 12.

During the working process, since the sensor 3 is arranged on the electrode 1 and sandwiched between the electrode 1 and the tissue of the inner wall of bronchus 13, the temperature of the electrode 1 and the tissue on the inner wall of bronchus 13 can be quickly monitored, so as to ensure the safety of the operation and the accuracy of the treatment. At the same time, the pressure between the electrode 1 and the tissue on the inner wall of bronchus 13 can also be monitored to determine whether the electrode 1 and the tissue on the inner wall of bronchus 13 are in good contact, so as to improve the ablation effect.

During the operation, by adjusting the parameters of the ablation radiofrequency, the thermal ablation treatment of different degrees of diseased tissue on the inner wall of bronchus 13 can be realized.

By rotating the handle 5, the ablation of the diseased tissue in different parts of the same segment of the inner wall of bronchus 13 can be realized.

By pushing the handle 5, the ablation of the diseased tissue in different segments of the inner wall of bronchus 13 can be achieved.

When it is necessary to perform nerve ablation on the left and right bronchus, or when multiple sets of separate control electrodes are used, the delivery tube 4 can be labelled. In this way, medical imaging techniques such as X-ray and Doppler ultrasound can be used to determine the location of the delivery tube 4.

In the radiofrequency ablation device provided in this embodiment, since the electrode 1 is not attached to the balloon 12 and there is a gap between the balloon catheter 42 and the inner tube 41, the balloon connector 50 can push the balloon 12 through the balloon catheter 42 to reach the target position of the balloon first. After then, the electrode 1 is delivered to the target position of the electrode, and then the balloon 12 is retracted to make it reach the target position of the electrode from the target position of the balloon, thereby making it enter the electrode 1. After the balloon 12 is inflated, the electrode 1 can be pushed further toward the inner wall of bronchus 13. Since the balloon 12 and the electrode 1 are delivered separately, it is convenient for the balloon 12 to pass through the bronchus. That is, for a bronchus of the same size, a balloon 12 with larger size is allowed to pass through, thereby increasing the support force of the balloon 12 on the electrode 1. At the same time, more coolant can be injected into the balloon 12 to improve the cooling effect on the electrode 1.

Obviously, the foregoing embodiments are only examples for the purpose of clear description, and are not intended to limit the manner of implementation. For a person of ordinary skill in the art, changes or modifications in other different forms can also be made based on the foregoing description. It is not necessary or possible to exhaust all implementations here. However, obvious changes or modifications derived therefrom still fall within the protection scope of the present invention.

## Claims

1. A radiofrequency ablation device, **characterized by** comprising:
an electrode (1) in a shape of a mesh, which has a contracted state that shrinks together under restriction and an expanded state that automatically expands outward without restriction, and is connected to an external electrical connector (51) through an electrode lead (44);
a balloon (12) located at the inner side of the electrode (1), which pushes against the electrode (1) when it is in an expanded state;
a delivery tube (4) comprising an outer tube (40) and an inner tube (41), the electrode lead (44) is located between the outer tube (40) and the inner tube (41), the delivery tube (4) further comprises a balloon catheter (42) communicating with one end of the balloon (12), which is located at the inner side of the inner tube (41) and has a gap with the inner tube (41).

2. The radio frequency ablation device according to claim 1, **characterized in that** the electrode (1) is formed by cutting a memory alloy.

3. The radio frequency ablation device according to claim 1, **characterized in that** the electrode (1) is provided with an ablation zone (11), which is located on a raised part (15) of the electrode (1).

4. The radiofrequency ablation device according to claim 3, **characterized in that** the raised part (15) comprises an end (10) of the electrode (1), and the ablation zone (11) is located on the end (10) of the electrode (1).

5. The radiofrequency ablation device according to claim 3, **characterized in that** a free end (17) of the electrode (1) extends outward.

6. The radiofrequency ablation device according to claim 5, **characterized in that** the distance between the free end (17) of the electrode (1) and the central axis of the electrode (1) is smaller than the distance between the raised part (15) and the center axis of the electrode (1) or smaller than a radius of a bronchus.

7. The radiofrequency ablation device according to claim 3, **characterized in that** the free end (17) of the electrode (1) is in a contracted state, and is provided with several anti-scratch structures (18).

8. The radiofrequency ablation device according to claim 7, **characterized in that** each of the anti-scratch structures (18) is provided with a small hole, and a connecting line passes through adjacent small holes in turn to form a spacing ring (19).

9. The radio frequency ablation device according to claim 1, **characterized in that** the balloon (12), when inflated, has a diameter greater than the diameter of the electrode (1) at the point where the balloon (12) abuts against the electrode (1), suitable for pushing the electrode (1) towards the inner wall of the bronchus (13).

10. The radio frequency ablation device according to claim 1, **characterized in that** the balloon (12) is provided with micropores (120) on its surface.

11. The radiofrequency ablation device according to claim 1, **characterized in that** the balloon catheter (42) is provided with an input cavity (42a) adapted to input coolant into the balloon (12), and an output cavity (42b) adapted to provide a passage for the coolant to flow out of the balloon (12).

12. The radiofrequency ablation device according to any one of claims 1 to 11, **characterized by** further comprising a sensor (3) arranged on the electrode (1), wherein the sensor (3) is connected to the electrical connector (51) through a sensor lead (45) which is located between the outer tube (40) and the inner tube (41).

13. The radiofrequency ablation device according to any one of claims 1 to 9, **characterized by** further comprising:
a negative plate (8);
a radiofrequency device (7), electrically connected to the negative plate (8) and connected to the electrical connector (51);
a pressure pump (9), connected to a balloon connector (50) through a first butt tube (60);
a circulating pump (6), connected to a coolant connector (52) through a second butt tube (61);
a bronchoscopy device, comprising a bronchoscope (2), an endoscopic visualization device (20) and a suction device (21), wherein the bronchoscope (2) has a working channel suitable for the passage of the delivery tube (4).
